# EUROPÄISCHE PATENTANMELDUNG

(11) **EP 2 495 009 A1**
(43) Veröffentlichungstag der Anmeldung: **05.09.2012**
(21) Anmeldenummer: 11001737.3
(22) Anmeldetag: 02.03.2011
(51) Int. Cl.: A61M 27/00, A61M 1/00

(54) **Wundversorgungsanordnung und Sauganschluss für eine Wundversorgungsanordnung**

(71) Anmelder: Lohmann & Rauscher GmbH, 2525 Schönau/Triesting (AT)
(72) Erfinder: Danei, Federico, 1180 Wien (AT); Wagner, Georg, 1080 Wien (AT); Rohrer, Christian, 4020 Linz (AT); Steinlechner, Erik, 2500 Baden (AT)
(74) Vertreter: Leinweber & Zimmermann

(57) **Zusammenfassung**

Die Erfindung betrifft eine Wundversorgungsanordnung mit einem zum Füllen der Wunde ausgelegten Füllmaterial und einem zum Absaugen von Wundexsudat aus dem Füllmaterial ausgelegten und auf der dem Wundgrund abgewandten Seite außerhalb des Füllmaterials anzuordnenden Sauganschluß mit einer zwischen dem Füllmaterial und dem Sauganschluß angeordneten Drainageschicht zum Einleiten des aus dem Füllmaterial abzusaugenden Exsudats in mindestens eine Absaugöffnung des Sauganschlusses.

## Beschreibung

Die Erfindung betrifft eine Wundversorgungsanordnung mit einem zum Füllen der Wunde ausgelegten Füllmaterial und einem zum Absaugen von Wundexsudat aus dem Füllmaterial ausgelegten und auf der dem Wundgrund abgewandten Seite außerhalb des Füllmaterials anzuordnenden Sauganschluß sowie einen Sauganschluß für eine solche Wundversorgungsanordnung.

Derartige Wundversorgungsanordnungen werden insbesondere im Rahmen der sog. Vakuumtherapie eingesetzt. Es hat sich gezeigt, daß im besonderen die Heilung chronischer Wunden durch Anlegen von Unterdruck an diese Wunden gefördert werden kann. Dabei hat es sich weiter als vorteilhaft erwiesen, wenn die Wunde mit einem offenporigen Schaum oder Gaze als Füllmaterial abgedeckt bzw. gefüllt wird und auf der dem Wundgrund abgewandten Seite des Füllmaterials ein Sauganschluß angelegt und mit einer zum Erzeugen von Unterdruck ausgelegten Saugeinrichtung verbunden wird. Dazu kann der Sauganschluß bspw. mit einem einerseits an einer bspw. in Form eines Rohrstutzens ausgeführten Verbindungseinrichtung des Sauganschlusses und andererseits an die Saugeinrichtung anschließbaren Schlauch ausgestattet sein. Zusätzlich kann eine bspw. als folienartiges Material ausgeführte Abdeckeinrichtung vorgesehen sein, die an die der Wunde benachbarte Hautfläche luftdicht angelegt wird und einen das Füllmaterial enthaltenden luftdicht abgeschlossenen Raum bildet, der über den Sauganschluß mit der Saugeinrichtung verbunden ist, um innerhalb dieses Raums einen Unterdruck zu erzeugen.

Wundversorgungsanordnungen, die im Rahmen der Vakuumtherapie einsetzbar sind, sind bspw. in der EP 0 620 720 B1 beschrieben. Der Offenbarungsgehalt dieser Schrift wird hinsichtlich der Einzelheiten der im Rahmen der Vakuumtherapie einsetzbaren schaumbildenden Mittel und der Saugeinrichtungen hiermit durch ausdrückliche Inbezugnahme in diese Beschreibung aufgenommen.

In der DE 10 2009 019 646 A1 ist eine zur Verbesserung des Exsudatmanagements zwischen dem Füllmaterial und dem Wundgrund einzulegende Kontaktschicht beschrieben, die einen Drainageraum zwischen dem Füllmaterial und dem Wundgrund bildet. Der Offenbarungsgehalt dieser Schrift wird hinsichtlich der Einzelheiten der den Drainageraum bildenden Kontaktschicht bzw. Wundabdeckung ausdrücklich in diese Beschreibung aufgenommen.

Im Rahmen der Vakuumtherapie einsetzbare Sauganschlüsse, welche über einen Schlauch mit einer Saugeinrichtung verbunden werden können, sind bspw. in der WO 03/073970 A1, der WO 2008/014358 A2 und der WO 2009/124548 A1 beschrieben. Ein als Saugkopf bezeichneter Sauganschluß mit zur Strömungsführung im Bereich der der Wunde zugewandten Begrenzungsfläche des Sauganschlusses dienenden Vorsprüngen ist in der EP 1 018 967 B1 beschrieben. Ferner ist ein Sauganschluß mit einer an das Füllmaterial anzulegenden Anlagefläche in Form einer scheibenartigen Schale in der EP 1 088 569 B1 angegeben. Bei einem in der WO 2010/008167 A2 beschriebenen Sauganschluß sind auf der dem Füllmaterial zugewandten Begrenzungsfläche durch Stege begrenzte Kanäle gebildet, durch die das Wundexsudat in Richtung auf eine Absaugöffnung geleitet werden soll. Schließlich ist in der EP 2 098 257 A1 ein Sauganschluß für den Einsatz in der Vakuumtherapie angegeben, welcher von einer Mehrzahl von Absaugkanälen durchsetzt ist, wobei ein zentraler Absaugkanal von einer Mehrzahl von Absaugkanälen mit kleinerem Querschnitt umgeben ist, die in den zentralen Absaugkanal münden.

In der DE 20 2010 005 872.4 ist ein Sauganschluß angegeben, bei dem eine dem Füllmaterial zugewandte Absaugöffnung von einem ringförmigen Vorsprung umlaufen wird.

Es hat sich gezeigt, daß bei Einsatz der bekannten Sauganschlüsse in vielen Fällen Probleme beim Abtransport von Wundexsudat beobachtet werden.

Angesichts dieser Probleme im Stand der Technik liegt der Erfindung die Aufgabe zugrunde, eine Wundversorgungsanordnung bereitzustellen, mit der zuverlässig ein zufriedenstellender Abtransport von Wundsekret aus dem Wundbereich möglich ist.

Erfindungsgemäß wird diese Aufgabe durch eine Weiterbildung der bekannten Wundversorgungsanordnungen gelöst, die im wesentlichen dadurch gekennzeichnet ist, daß zwischen dem Füllmaterial und dem Sauganschluß eine Drainageschicht zum Einleiten des aus dem Füllmaterial abzusaugenden Exsudats in mindestens eine Absaugöffnung des Sauganschlusses vorgesehen ist.

Dabei geht die Erfindung auf die Erkenntnis zurück, daß die bei den bekannten Sauganschlüssen auf der dem Füllmaterial zugewandten Seite vorgesehenen Vorsprünge oder Stege den Abtransport von Wundexsudat aus dem Füllmaterial dadurch behindern, daß sie sich unter der Wirkung des Differenzdrucks zwischen der dem Füllmaterial abgewandten äußeren Begrenzungsfläche des Sauganschlusses und der dem Füllmaterial zugewandten Begrenzungsfläche des Sauganschlusses in das Füllmaterial eingraben und so die den Transport des Wundexsudats ansonsten begünstigenden Eigenschaften des bspw. als offenporiger Schaum oder Gaze ausgeführten Füllmaterials gerade im Bereich des Sauganschlusses beeinträchtigen.

Bei Sauganschlüssen gemäß der EP 2 098 257 A1 wird das Problem beobachtet, daß die Saugkanäle mit geringem Querschnitt leicht verstopfen können, falls es zu der Koagulation von Wundexsudat in Form von Gerinnseln kommt.

Diese Probleme werden bei Einsatz der erfindungsgemäßen Wundversorgungsanordnungen gelöst, weil die dem Wundgrund zugewandte Anlagefläche des Sauganschlusses nicht mehr unmittelbar an das Füllmaterial angelegt wird, sondern an die Drainageschicht, über die das vom Füllmaterial aufgenommene Wundexsudat der Absaugöffnung zugeleitet wird.

Bei der erfindungsgemäßen Wundversorgungsanordnung sind daher weder kanalbildende Vorsprünge an der dem Füllmaterial zugewandten Begrenzungsfläche des Absauganschlusses noch eine Erweiterung der Saugfläche in Form einer scheibenartigen Schale erforderlich. Vielmehr reicht es aus, wenn die dem Füllmaterial zugewandte Begrenzungsfläche des Absauganschlusses flanschartig eben ausgeführt ist und von der Saugöffnung durchsetzt wird, weil die Einleitung des Wundexsudats aus dem Füllmaterial nicht durch eine besondere Beschaffenheit des Anschlusses, sondern durch die zwischen dem Anschluß und dem Füllmaterial angeordnete Drainageschicht bewirkt wird. Wenn die an die Drainageschicht anzulegende Anlagefläche des Sauganschlusses eben, d.h. ohne Vorsprünge, sei es in Form von Kanäle begrenzenden Stegen, sei es in Form von ringförmigen Vorsprüngen wie bei den scheibenartigen Schalen gemäß EP 1 088 569 B1, ausgeführt ist, wird das Eindrücken vorspringender Strukturen in die Drainageschicht verhindert und damit eine zuverlässige Funktion der Drainageschicht gewährleistet.

Ähnlich wie bei herkömmlichen Sauganschlüssen kann auf der der Anlagefläche abgewandten Seite des erfindungsgemäßen Sauganschlusses eine Verbindungseinrichtung, wie etwa ein Rohrstutzen, vorgesehen sein, mit dem eine luftdichte Verbindung zwischen der dem Füllmaterial zugewandten Absaugöffnung und einem auf der dem Füllmaterial abgewandten Seite außerhalb des Füllmaterials angeordneten Absaugschlauch hergestellt werden kann.

Im Sinne einer besonders einfachen Anwendung erfindungsgemäßer Wundversorgungsanordnungen hat es sich als zweckmäßig erwiesen, wenn die Drainageschicht an der Anlagefläche befestigt, insbesondere auf die Anlagefläche aufgeklebt, aufgeschweißt, an die Anlagefläche geklammert und/oder genäht ist. Die Gesamtanordnung, bestehend aus Sauganschluß und Drainageschicht, kann dann als Ganzes an der gewünschten Stelle auf dem Füllmaterial positioniert werden.

Überraschenderweise hat es sich gezeigt, daß bei der Vakuumtherapie ein gleichzeitig mit dem Erzeugen des Unterdrucks erfolgender kontinuierlicher Lufteintritt in den Wundbereich den Heilungsvorgang weiter verbessert. Daher weist eine Wundversorgungsanordnung gemäß einer besonders bevorzugten Ausführungsform der Erfindung einen Sauganschluß auf, welcher neben einer zum Absaugen des Exsudats verwendeten Absaugöffnung auch noch eine insbesondere in dem flanschartigen Anlagebereich des Sauganschlusses angeordnete und zur Belüftung der Wunde dienende Belüftungsöffnung aufweist. Ein durch die Belüftungsöffnung möglicher kontinuierlicher Lufteintritt in den Wundbereich verursacht einen kontrollierten und kontinuierlichen Druckabfall bei Verbinden einer Saugeinrichtung mit der Absaugöffnung. Dadurch kann die Entfernung des Exsudats weiter verbessert werden. Die zum Absaugen des Exsudats verwendete Pumpe erzeugt dann einen höheren Durchfluß und eine verbesserte Saugwirkung. Sie tritt öfter in Aktion und saugt mehr ab.

Zur Vermeidung einer Kontamination der Wunde ist der Belüftungsöffnung zweckmäßigerweise ein antibakterielles Filter zugeordnet, welches in der Belüftungsöffnung angeordnet sein oder die Belüftungsöffnung abdecken kann. Das Filter ist zweckmäßigerweise hydrophob und weist zum Erhalt der gewünschten Filterwirkung eine Porengröße von 0,001, insbes. 0,005, bevorzugt 0,02, besonders bevorzugt 0,1 bis 5 Mikrometer auf. Bei einer Porengröße von weniger als 0,001 Mikrometer wird die gewünschte Belüftung beeinträchtigt. Bei einer Porengröße von mehr als 5 Mikrometer wird die antibakterielle Wirkung kaum noch erzielt. Das Material des Filters kann insbesondere Polytetrafluorethylen enthalten. Durch Wahl der Porengröße des Filters wird auch der Luftdurchfiuß im Wundbereich beeinflußt, wobei eine Verkleinerung der Poren zu einer Verminderung des Lufteintritts führt.

Zusätzlich oder alternativ zu der Belüftungsöffnung kann der Wundversorgungsanordnung auch einen der Absaugöffnung zugewandten Multilumenschlauch aufweisen, der insbesondere drei Lumen enthalten kann, von denen nur einer zum Absaugen des Exsudats, ein anderer zur kontrollierten Luftzufuhr und ein dritter zur Druckmessung direkt an der Wunde benutzt wird. Durch Verwendung des Multilumenschlauchs kann die Saugwirkung verbessert werden, ohne daß der Sauganschluß bauliche Änderungen benötigt. Allerdings wird die Saugwirkung bei Verwendung einer zusätzlichen Belüftungsöffnung noch weiter verbessert. Eine weitere Verbesserung ist erreichbar, wenn der als Verbindungseinrichtung dienende Rohrstutzen eine dem Multilumenschlauch entsprechende Anzahl an Lumen aufweist.

Im Sinne einer optimalen Wundversorgung ohne Entfernen einer erfindungsgemäßen Wundversorgungsanordnung hat es sich als zweckmäßig erwiesen, wenn der Sauganschluß zusätzlich zu der Absaugöffnung auch noch eine zum Zuführen eines Wundversorgungsprodukts ausgelegte und vorzugsweise den Anlagebereich durchsetzende Zuführöffnung aufweist, der bei einer besonders bevorzugten Ausführungsform der Erfindung auf der der Anlagefläche abgewandten Seite des Sauganschlusses eine weitere Verbindungseinrichtung, wie etwa ein weiterer Rohrstutzen, zum Herstellen einer Verbindung zwischen der Zuführöffnung und einem Zuführschlauch zugeordnet sein kann. Über die Zuführöffnung kann bspw. eine Spüllösung ggf. mit Medikamenten, Desinfektionsmitteln und dgl. in den Wundbereich eingebracht werden. Die Zuführöffnung kann ebenso wie die Absaugöffnung von der Drainageschicht abgedeckt sein. Allerdings ist es im Rahmen der Erfindung besonders bevorzugt, wenn diese Öffnung nicht von der Drainageschicht abgedeckt ist, um das Diffundieren der Spüllösung in den Wundbereich zu verbessern.

Zur weiteren Verbesserung des Exsudatmanagements kann die erfindungsgemäße Wundversorgungsanordnung eine zwischen dem Wundgrund und dem Füllmaterial angeordnete, eine wundseitige Drainage bewirkende Kontaktschicht aufweisen.

Im Rahmen der Erfindung hat es sich als besonders vorteilhaft erwiesen, wenn die Drainageschicht und/oder die Kontaktschicht, entsprechend den Wundabdeckungen gemäß der DE 10 2009 019 646 A1, zwei etwa parallel zueinander verlaufende bahnförmige Elemente aufweist, zwischen denen ein Drainageraum gebildet ist, dessen Tiefe in einer sich etwa senkrecht zu den bahnförmigen Elementen erstreckenden Tiefenrichtung eine Kapillarwirkung auf in dem Drainageraum aufgenommene Exsudate gewährleistet. Die Tiefe des Drainageraums kann dazu 5 mm oder weniger und 0,5 mm oder mehr betragen. Dabei weist jedes der bahnförmigen Elemente zweckmäßigerweise eine den Durchtritt von Körperflüssigkeit in den Drainageraum ermöglichende Öffnung auf, wobei mindestens eine Öffnung durch einen sich ausgehend von einem der bahnförmigen Elemente in Richtung auf die gegenüberliegende innere Begrenzungsfläche des anderen bahnförmigen Elements erstreckenden und in den Drainageraum mündenden Kanal gebildet ist, dessen Kanalwand einstückig mit dem bahnförmigen Element ausgeführt, insbesondere durch Perforation des bahnförmigen Elements gebildet ist.

Im Sinne einer besonders ausgeprägten Kapillarwirkung hat es sich als günstig erwiesen, wenn sich die Querschnittsfläche des Kanals in einer sich senkrecht zur Tiefenrichtung erstreckenden Ebene, ausgehend von dem bahnförmigen Element, in Richtung auf die gegenüberliegende andere Begrenzungsfläche, insbesondere zum Erhalt einer den Eintritt von Körperflüssigkeit in den Drainageraum begünstigenden Kapillarwirkung, verringert. Dabei kann mindestens ein bahnförmiges Element eine Vielzahl von vorzugsweise rasterförmig angeordneten Öffnungen aufweisen, wobei der Abstand zwischen benachbarten Öffnungen 15 mm oder weniger, vorzugsweise 5 mm oder weniger, insbesondere 3 mm oder weniger, beträgt und die Mündungen der in einem bahnförmigen Element angeordneten Öffnungen in einer Projektion längs der Tiefenrichtung zwischen den Mündungen der in dem anderen bahnförmigen Element angeordneten Öffnungen angeordnet sind, wobei sich mindestens ein Kanal in Tiefenrichtung über 50 % oder mehr der gesamten Tiefe des Drainageraums erstreckt.

Die Kanalwand der die Öffnung bildenden Kanäle ist in einer sich parallel zur Tiefenrichtung erstreckenden Schnittebene zumindest abschnittweise bogenförmig ausgeführt und geht stetig in die Begrenzungsfläche des bahnförmigen Elements über. Weitere Merkmale erfindungsgemäß einsetzbarer Kontakt- und/oder Drainageschichten sind in der DE 10 2009 019 646 A1 angegeben, deren Offenbarungsgehalt hiermit durch ausdrückliche Inbezugnahme in diese Beschreibung aufgenommen wird.

Wie der Erläuterung bekannter Wundversorgungsanordnungen zu entnehmen ist, weist auch die erfindungsgemäße Wundversorgungsanordnung zum Einsatz in der Vakuumtherapie zweckmäßigerweise eine an der die Wunde umgebenden Haut luftdicht festlegbare und zur Herstellung eines das Füllmaterial enthaltenden luftdicht abgeschlossenen Raums dienende Abdeckeinrichtung, wie etwa eine Abdeckfolie, auf.

Wenn die Drainageschicht die Anlagefläche des Sauganschlusses nur teilweise bedeckt, wobei vorzugsweise zumindest die Absaugöffnung und das Filter abgedeckt sind, und ein die Drainageschicht umlaufender Befestigungsbereich an der Anlagefläche des Sauganschlusses vorgesehen ist, kann der Sauganschluß mit diesem Befestigungsbereich auf die Abdeckeinrichtung aufgeklebt werden. Dazu kann der Befestigungsbereich des Sauganschlusses mit einem geeigneten Klebstoff versehen sein. Alternativ kann statt Klebstoff auch etwa ein doppelseitiges Klebeband verwendet werden. Der Klebstoff (z. B. Acrylat, Silikon, Polyurethan) kann dabei teilweise (z. B. in Ringen), porös oder vollflächig aufgebracht sein. Auch das Klebeband kann teilweise (z. B. in Ringen) oder vollflächig angebracht sein. Das doppelseitige Klebeband kann außerdem auf beiden Seiten mit dem selben Klebstoff beschichtet sein (z. B. Acrylat, Silikon, Polyurethan), oder die zwei Seiten können mit zwei verschiedenen Klebstoffen beschichtet sein (insbes. auf der oberen Seite, in Kontakt mit der Anlagefläche 12, mit Silikonkleber und auf der unteren Seite mit Acrylatkleber). Sowohl der Klebstoff als auch das Klebeband können mit einer lösbaren Schutzschicht versehen sein.

Wenn die Drainageschicht die Anlagefläche des Sauganschlusses vollständig bedeckt, kann die bspw. folienartig ausgeführte Abdeckeinrichtung auf einem der Anlagefläche abgewandten und die bspw. in Form eines Rohrstutzens ausgeführte Verbindungseinrichtung des Sauganschlusses umlaufenden Befestigungsbereich des Sauganschlusses aufgeklebt sein.

Ebenso wie bei bekannten Wundversorgungsanordnungen zum Einsatz in der Vakuumtherapie kann das Füllmaterial erfindungsgemäßer Wundversorgungsanordnungen einen offenporigen Schaum oder Gaze aufweisen.

Ein zum Einsatz in einer erfindungsgemäßen Wundversorgungsanordnung ausgelegter erfindungsgemäßer Sauganschluß ist im wesentlichen dadurch gekennzeichnet, daß er eine im wesentlichen ebene Anlagefläche aufweist, mit der er an die Drainageschicht anlegbar ist, so daß das Eindringen von Vorsprüngen in die Drainageschicht und/oder das Füllmaterial sicher ausgeschlossen ist. Dabei wird die Anlagefläche von einer Saugöffnung durchsetzt, welche mit einer Verbindungseinrichtung in Form eines Rohrstutzens auf der der Wunde abgewandten Seite des Anschlusses verbunden ist, um so einen luftdichten Anschluß eines Absaugschlauchs zu gewährleisten. Über den Absaugschlauch kann mit Hilfe einer Saugeinrichtung Exsudat über die Absaugöffnung in der Anlagefläche abgesaugt und ein Unterdruck in dem von dem Füllmaterial ausgefüllten Raum erzeugt werden, um so die Wundheilung zu begünstigen.

Wie vorstehend bereits erläutert, kann die Drainageschicht einer erfindungsgemäßen Wundversorgungsanordnung auf einer Anlagefläche des erfindungsgemäßen Sauganschlusses befestigt, insbesondere aufgeklebt, aufgeschweißt, an den Sauganschluß geklammert und/oder genäht sein.

Gemäß einer besonders bevorzugten Ausführungsform der Erfindung weist der Sauganschluß einen von einer Absaugöffnung durchsetzten, insbesondere flanschartigen Anlagebereich auf, der zusätzlich auch noch von einer Belüftungsöffnung und/oder einer zum Zuführen eines Wundversorgungsprodukts dienenden Zuführöffnung durchsetzt ist, wobei der Zuführöffnung eine weitere, insbesondere nach Art eines Rohrstutzens ausgeführte Verbindungseinrichtung zum Herstellen einer Verbindung zwischen der Zuführöffnung und einem Zuführschlauch zugeordnet sein kann.

Nachstehend wird die Erfindung unter Bezugnahme auf die Zeichnung, auf die hinsichtlich aller erfindungswesentlichen und in der Beschreibung nicht näher herausgestellten Einzelheiten ausdrücklich verwiesen wird, erläutert. In der Zeichnung zeigt:
- **Fig. 1**: einen erfindungsgemäßen Sauganschluß gemäß einer ersten Ausführungsform der Erfindung,
- **Fig. 2**: einen erfindungsgemäßen Sauganschluß gemäß einer zweiten Ausführungsform der Erfindung,
- **Fig. 3**: eine erfindungsgemäße Wundversorgungsanordnung gemäß einer ersten Ausführungsform der Erfindung,
- **Fig. 4**: eine erfindungsgemäße Wundversorgungsanordnung gemäß einer zweiten Ausführungsform der Erfindung,
- **Fig. 5**: einen Sauganschluß gemäß einer dritten Ausführungsform der Erfindung,
- **Fig. 6**: eine mit dem Sauganschluß gemäß Fig. 5 ausgeführte Wundversorgungsanordnung,
- **Fig. 7**: einen Sauganschluß gemäß einer vierten Ausführungsform der Erfindung,
- **Fig. 8**: eine Wundversorgungsanordnung mit einem Sauganschluß gemäß Fig. 7,
- **Fig. 9**: einen Sauganschluß gemäß einer fünften Ausführungsform der Erfindung,
- **Fig. 10**: eine Wundversorgungsanordnung mit einem Sauganschluß gemäß Fig. 9, und
- **Fig. 11**: einen Sauganschluß gemäß einer sechsten Ausführungsform der Erfindung.

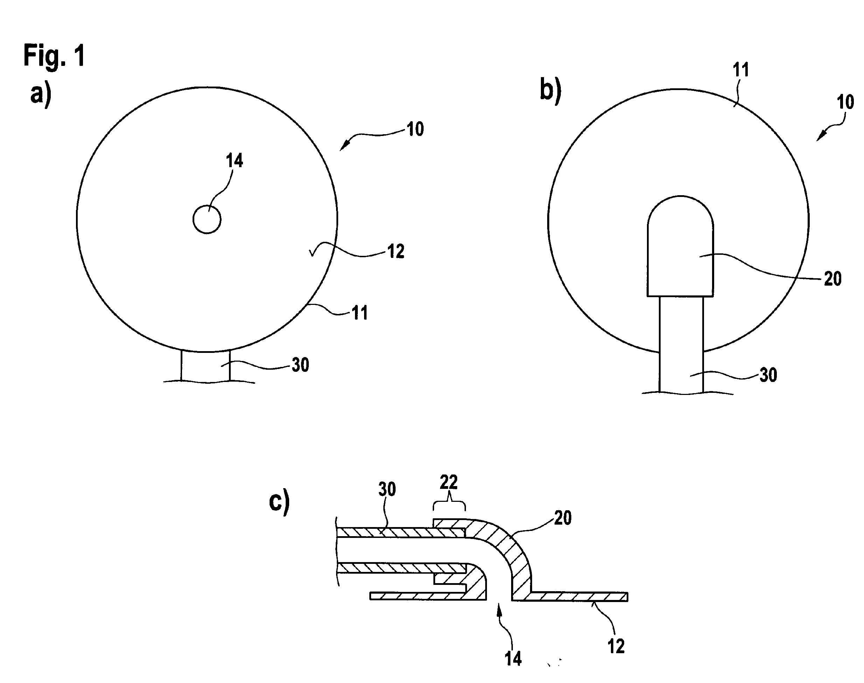

In Fig. 1a) ist eine Ansicht eines erfindungsgemäßen Sauganschlusses 10 von unten, in Fig. 1b) eine Ansicht des Sauganschlusses 10 gemäß Fig. 1a) von oben und in Fig. 1 c) eine Schnittdarstellung eines erfindungsgemäßen Sauganschlusses 10 gezeigt.

Der in Fig. 1 dargestellte Sauganschluß 10 weist einen flanschartigen Anlagebereich 11 mit einer kreisscheibenförmigen, ebenen Anlagefläche 12 ohne Vorsprünge auf. Die Anlagefläche 12 ist von einer Absaugöffnung 14 durchsetzt. Dabei ist die ebenfalls kreisförmige Absaugöffnung 14 im Zentrum der Anlagefläche 12 angeordnet. Die Absaugöffnung 14 mündet in eine Verbindungseinrichtung 20, welche nach Art eines Rohrstutzens ausgeführt ist und eine Umlenkung der senkrecht zur Anlagefläche 12 ausgerichteten Exsudatströmung um 90° bewirkt, so daß die Strömung nach Umlenkung etwa parallel zur Anlagefläche 12 ausgerichtet ist. Der Rohrstutzen 20 weist an seinem der Absaugöffnung 14 abgewandten Ende einen Anschlußbereich 22 mit erweitertem Innendurchmesser auf, in den ein Absaugschlauch 30 luftdicht einführbar ist. Durch die Umlenkung der Absaugströmung mit Hilfe des Rohrstutzens 20 wird eine Ausrichtung des Absaugschlauchs 30 in einer parallel zur Anlagefläche 12 verlaufenden Richtung ermöglicht. Dadurch wird eine störungsarme Anlage des Sauganschlusses 10 und des damit verbundenen Absaugschlauchs 30 an dem Körper des Patienten ermöglicht.

Bei der in Fig. 1c) dargestellten Ausführungsform ist der Absaugschlauch 30 so dimensioniert, daß seine innere Begrenzungsfläche mit einem an den Anschlußbereich 22 angrenzenden Innenflächenbereich des Rohrstutzens 20 fluchtet, um so den Strömungswiderstand für das Wundexsudat zu minimieren.

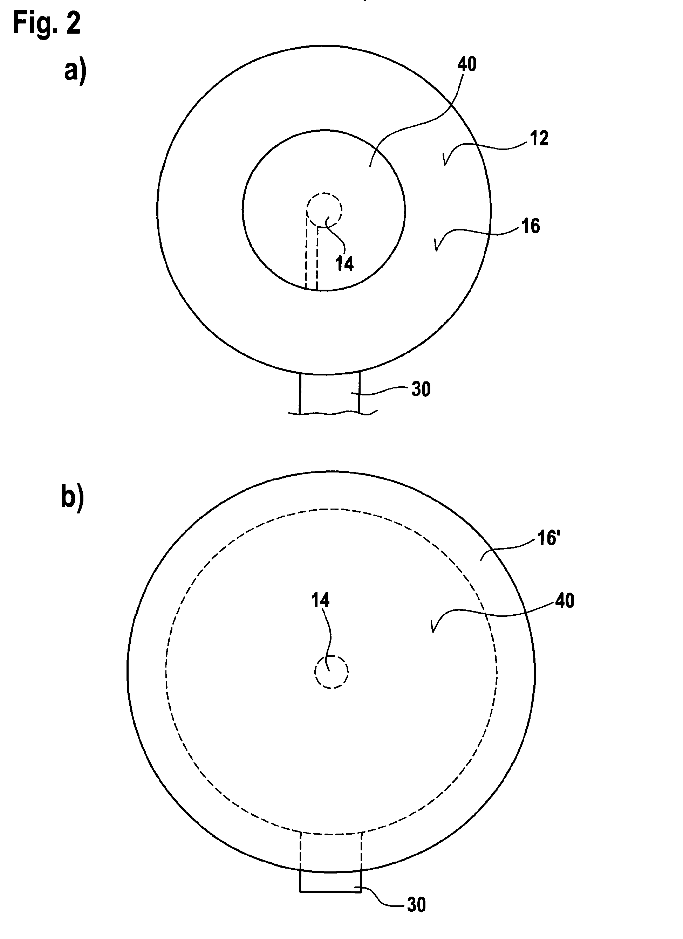

Bei der Ausführungsform gemäß Fig. 2a) ist ein die Absaugöffnung 14 enthaltender zentraler Bereich der Anlagefläche 12 von einer zum Einleiten von Wundexsudat in die Absaugöffnung 14 ausgelegten Drainageschicht 40 abgedeckt. Die Drainageschicht 40 ist auf die Anlagefläche 12 aufgeklebt und wird von einem Befestigungsbereich 16 der Anlagefläche 12 ringförmig umlaufen.

Bei der Ausführungsform gemäß Fig. 2b) ist die Anlagefläche 12 vollständig von der Drainageschicht 40 abgedeckt. Auf der der Anlagefläche 12 abgewandten Begrenzungsfläche des Sauganschlusses 10 ist ein den Rohrstutzen 20 umlaufender Befestigungsbereich 16' vorgesehen, welcher ebenso wie der Befestigungsbereich 16 mit einer Klebeschicht versehen sein kann, die vor Benutzung des Sauganschlusses 10 in einer erfindungsgemäßen Wundversorgungsanordnung von einer lösbaren Schutzschicht abgedeckt sein kann.

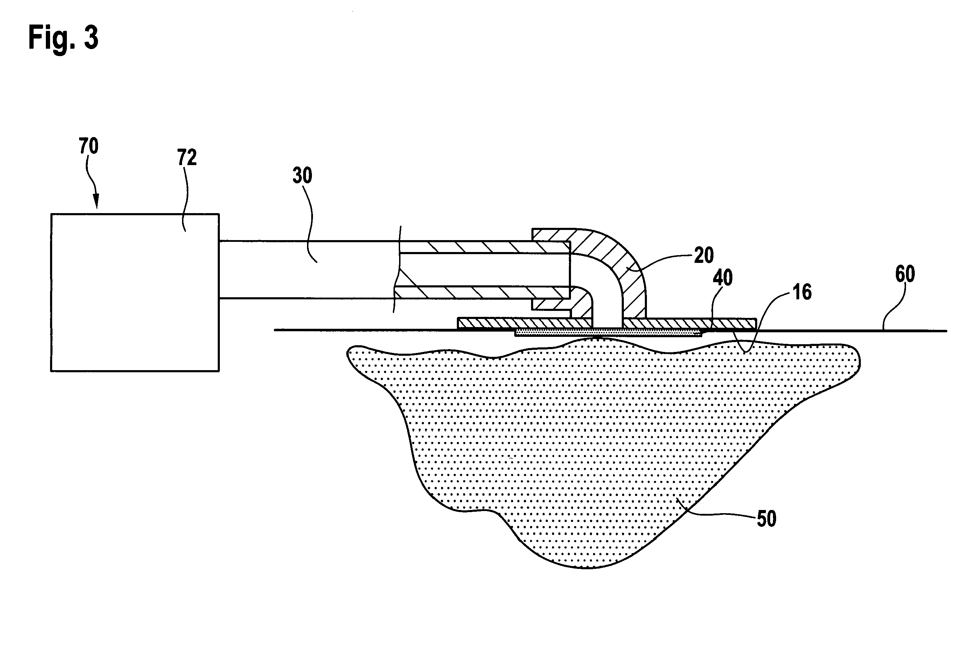

Fig. 3 zeigt den Einsatz eines Sauganschlusses 10 gemäß Fig. 2a) in einer erfindungsgemäßen Wundversorgungsanordnung, die ein eine Wunde ausfüllendes Füllmaterial 50 aufweist, wobei eine Abdeckfolie 60 an der die Wunde umgebenden Haut aufgeklebt ist. Die Abdeckfolie 60 weist eine zentrale Öffnung auf, welche von der auf die Anlagefläche 12 aufgeklebten Drainageschicht 40 durchsetzt wird, so daß das Wundexsudat aus dem Füllmaterial 50 über die Drainageschicht 40 in den Rohrstutzen 20 eingeleitet und über den Absaugschlauch 30 mit einer Saugeinrichtung 70 in einen Sammelbehälter 72 abgesaugt werden kann.

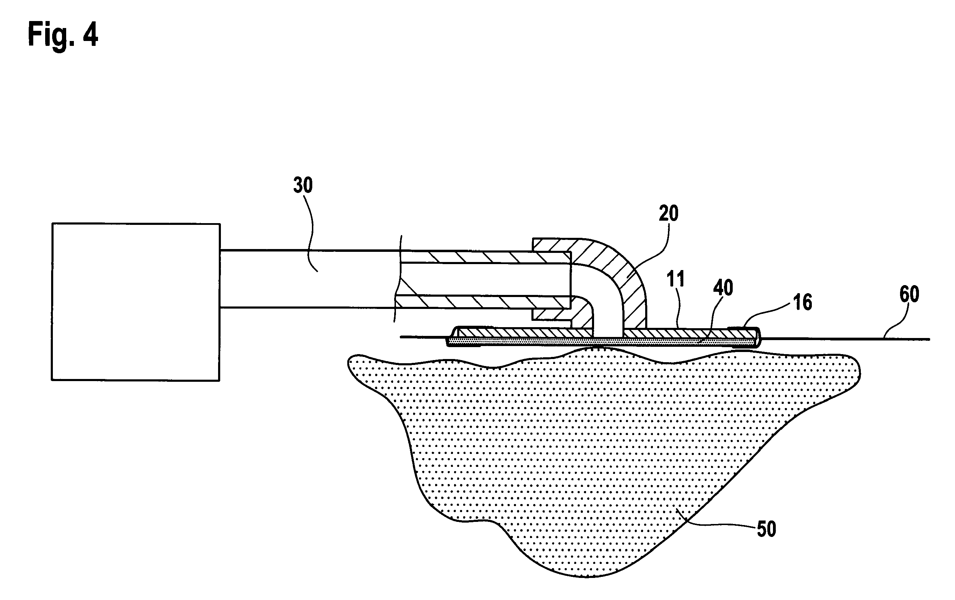

Die Ausführungsform gemäß Fig. 4 unterscheidet sich von der Ausführungsform gemäß Fig. 3 dadurch, daß der Sauganschluß 10 gemäß Fig. 2b) zum Einsatz kommt und die Abdeckfolie 60 auf der dem Füllmaterial 50 abgewandten Begrenzungsfläche und dem den Rohrstutzen 20 ringförmig umlaufenden Befestigungsbereich 16 aufgeklebt ist. Zusätzlich kann ein die Absaugöffnung 14 umlaufender Randbereich der Drainageschicht 40 auf einen Befestigungslappen der Abdeckfolie 60 aufgeklebt sein, so daß ein die Drainageschicht 40 und den Sauganschluß 10 aufweisender Rand in einer Tasche der Abdeckfolie 60 aufgenommen ist. Dadurch wird eine besonders luftdichte Verbindung des Sauganschlusses 10 mit der Abdeckfolie 60 erreicht.

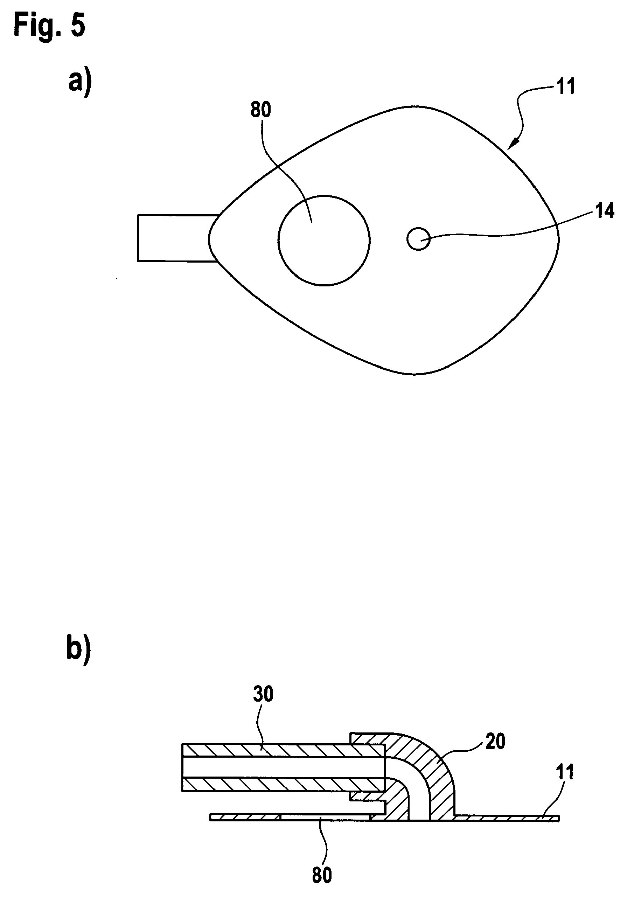

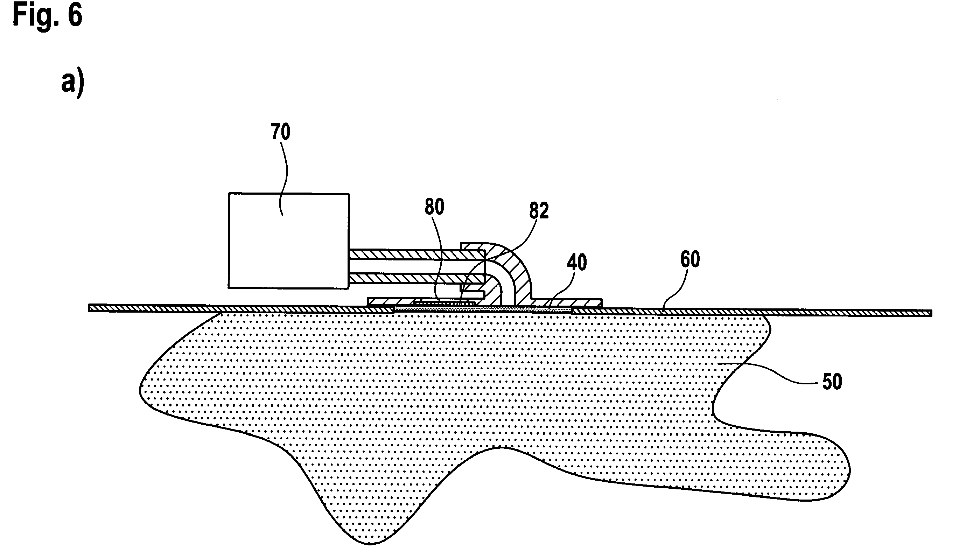

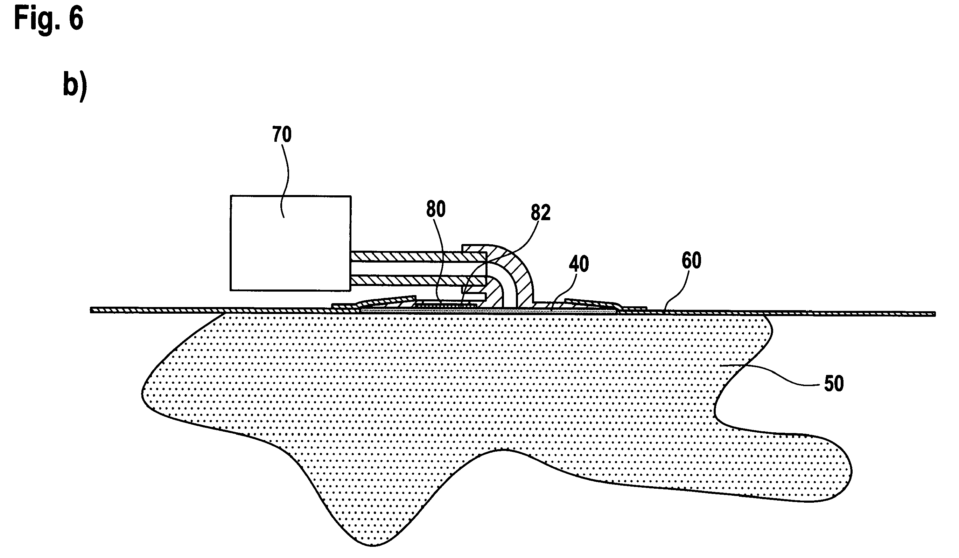

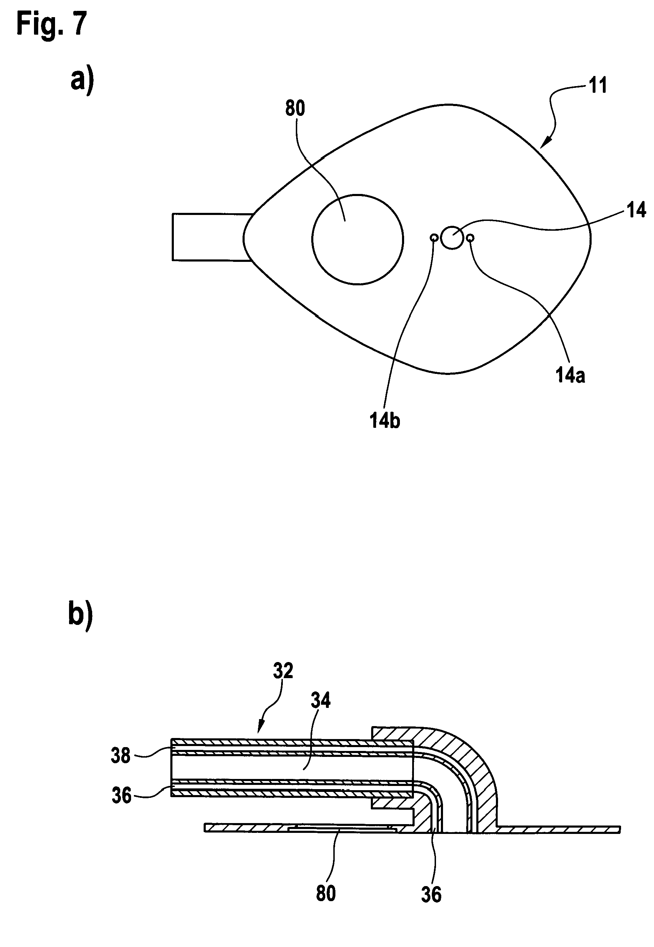

Der Sauganschluß gemäß Fig. 5 unterscheidet sich im wesentlichen dadurch von dem Sauganschluß gemäß den Fig. 1 und 2, daß der Anlagebereich 11 zusätzlich zu einer Ansaugöffnung 14 auch noch eine Belüftungsöffnung 80 aufweist, durch die die Wunde kontrolliert belüftet werden kann, um so die Strömungsverhältnisse im Wundbereich im Sinne einer Verbesserung der Exsudatabführung zu verbessern. Die Belüftungsöffnung 80 weist einen größeren Durchmesser auf als die Absaugöffnung 14. Wie besonders deutlich der Darstellung in Fig. 5b zu entnehmen ist, weist der die Belüftungsöffnung 80 in dem Anlagebereich 11 umlaufende Rand einen Absatz auf, der gemäß Fig. 6 und 7 als Auflagefläche für ein antibakterielles Filter 82 eingesetzt werden kann. Dadurch wird der Strömungswiderstand im Bereich der Belüftungsöffnung 80 erhöht, was allerdings durch Einstellung des Durchmessers der Belüftungsöffnung 80 wieder ausgeglichen werden kann. Im Rahmen der Erfindung kann der Strömungswiderstand des Filters 82 ausgenutzt werden, um die Luftströmung zu steuern. Dabei wird der Strömungswiderstand durch Verkleinerung der Filterporen vergrößert. Diese Steuerung begünstigt die Erzeugung des Unterdrucks bei gleichzeitiger Belüftung. Sofern die Belüftungsöffnung 80 zu groß gewählt wird, kann der Unterdruck ohne Einsatz eines Filters nicht erzeugt werden. Die Belüftungsöffnung 80 kann auch über der Abdeckfolie 60 angeordnet sein (in der Draufsicht). Wesentlich ist, daß der Wunde Luft zugeführt wird. Dazu kann es zweckmäßig sein, etwas versetzt ein Loch in der Abdeckfolie 60 vorzusehen, damit die Luft ungehindert zur Belüftungsöffnung 80 strömen kann.

Der Drainageraum der Drainageschicht 40 kann genügen, um Luft in die zentrale Öffnung einströmen zu lassen, auch wenn die Belüftungsöffnung 80 auf der dem Füllmaterial 50 zugewandten Seite mit der Abdeckfolie 60 abgedeckt ist.

Das antibakterielle Filter 82 ist bei der in der Zeichnung dargestellten Ausführungsform aus Polytetrafluorethylen gebildet und weist eine Porengröße im Bereich von 0,001, insbes. 0,005, bevorzugt 0,02, besonders bevorzugt 0,1 bis 5 Mikrometern auf. Es wird bei der Wundversorgungsanordnung gemäß Fig. 6a und 6b von der Drainageschicht 40 abgedeckt. Wie anhand der Fig. 6a und 6b erläutert, kann der Sauganschluß gemäß Fig. 5 ebenso wie der Sauganschluß gemäß Fig. 1 entsprechend den Darstellungen in den Fig. 3 und 4 angebracht werden, so daß er entweder auf einer Abdeckfolie 60 aufgeklebt ist oder von der Abdeckfolie 60 übergriffen wird. Wesentlich ist, daß die Belüftungsöffnung 80 auf der dem Füllmaterial 50 abgewandten Seite nicht von der Abdeckfolie 60 abgedeckt ist. Wenn die Belüftungsöffnung 80 bzw. das Filter 82 auf der dem Füllmaterial 50 zugewandten Seite von der Abdeckfolie 60 abgedeckt ist, kann die Luft durch den zwischen zwei Schichten der Drainagefolie gebildeten Raum dennoch in den Wundbereich einströmen.

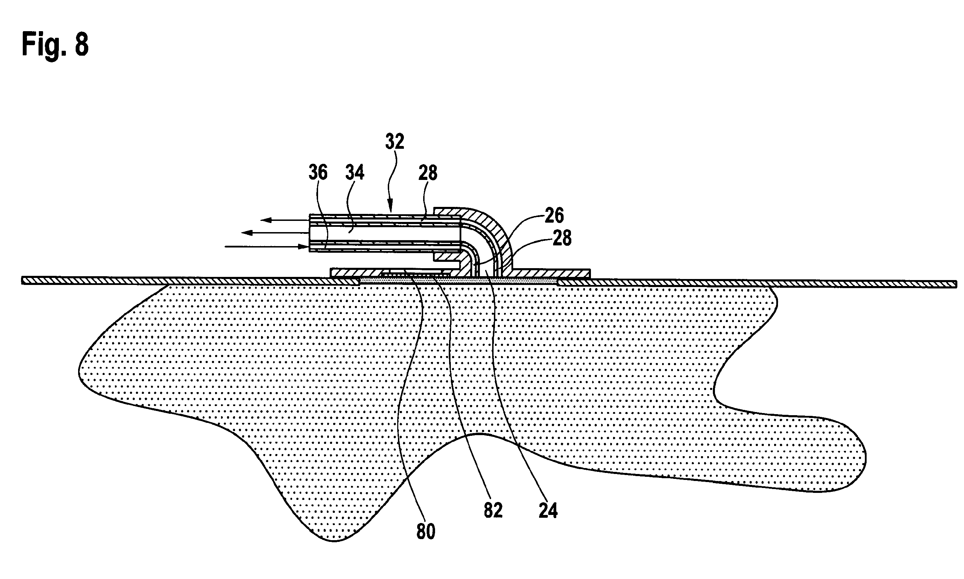

Die Ausführungsform gemäß Fig. 7 unterscheidet sich im wesentlichen dadurch von der Ausführungsform gemäß Fig. 5, daß der Absaugschlauch als Dreilumenschlauch 32 verwirklicht ist, wobei wie in Fig. 8 schematisch angedeutet ist, das mittlere und größte Lumen 34 zum Erzeugen des Unterdrucks im Wundbereich benutzt wird, ein kleines Lumen 36 zum Belüften der Wunde benutzt werden kann und ein weiteres kleineres Lumen 38 zum Messen des Drucks im Wundbereich gedacht ist. Der Anschlußbereich 22 weist ebenso den Lumen 32, 34 und 36 entsprechende Lumen 24, 26 und 28 auf.

Die Ausführungsform gemäß den Fig. 7 und 8 weist neben dem Belüftungslumen 26 auch noch eine Belüftungsöffnung 80 auf, um das Exsudat-Management weiter verbessern zu können.

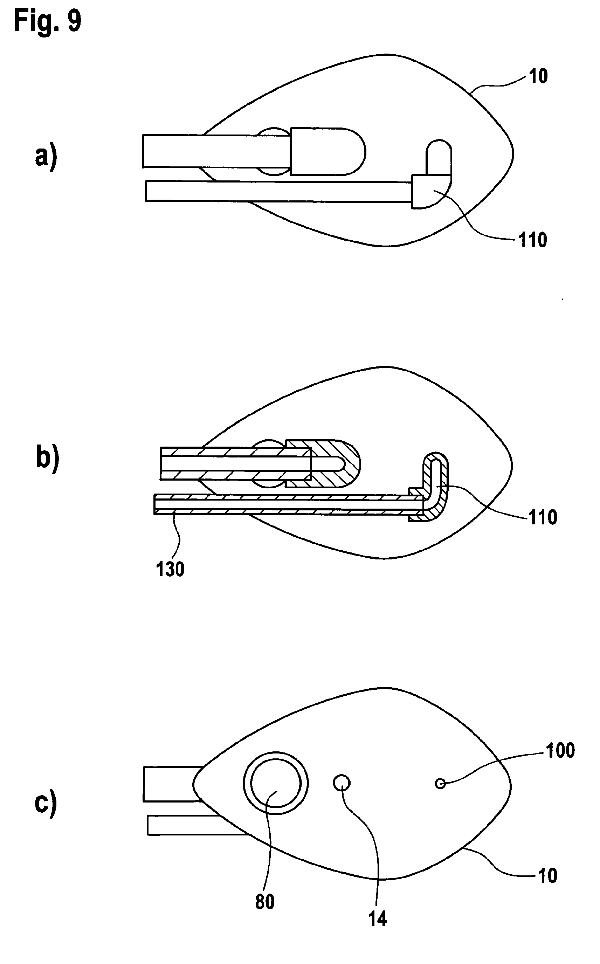

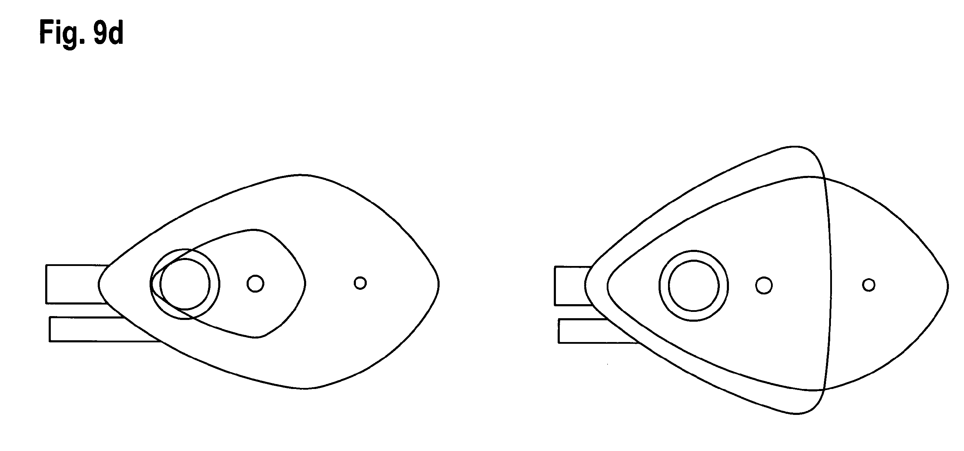

Die Ausführungsform gemäß Fig. 9 unterscheidet sich im wesentlichen dadurch von der Ausführungsform gemäß Fig. 5, daß zusätzlich zu der Absaugöffnung 14 und der Belüftungsöffnung 80 auch noch eine Zuführöffnung 100 in den Anlagebereich 11 des Sauganschlusses 10 vorgesehen ist, der ein weiterer Rohrstutzen 110 zum Verbinden der Zuführöffnung 100 mit einem weiteren Schlauch 130 zugeordnet ist. Über den Schlauch 130, den weiteren Rohrstutzen 110 und die Zuführöffnung 100 können Wundversorgungsmittel wie etwa eine ggf. auch mit Medikamenten, Desinfektionsmitteln oder dgl. versetzte Spüllösung, in den Wundbereich eingebracht werden. Die Ausführungsform gemäß Fig. 9 kann auch ohne Verwendung einer separaten Belüftungsöffnung 80 benutzt werden, weil die Zuführöffnung 100 auch zum Belüften eingesetzt werden kann. Im Sinne eines guten ExsudatManagements hat es sich allerdings als besonders zweckmäßig erwiesen, wenn sowohl eine Belüftungsöffnung 80 als auch eine Zuführöffnung 100 neben der Absaugöffnung 14 in dem Anlagebereich 11 des Sauganschlusses 10 vorgesehen sind.

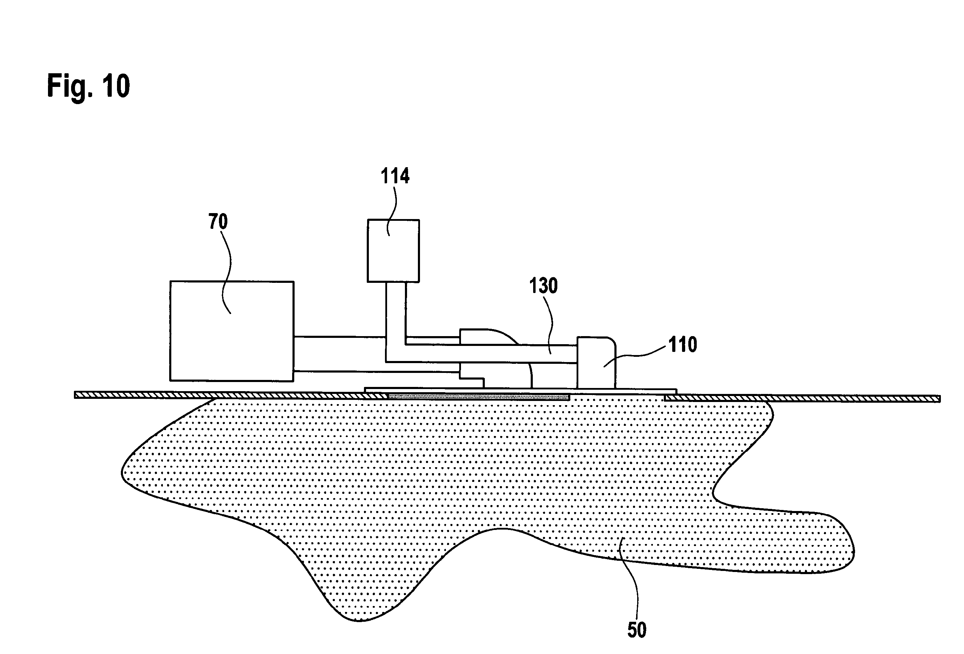

Wie in Fig. 10 erkennbar ist, kann die Spüllösung über den weiteren Schlauch 130 und den weiteren Rohrstutzen 110 mit Hilfe eines entsprechenden Dosierelements 114 in den Bereich der Wunde eingebracht werden. Dabei hat es sich als besonders zweckmäßig erwiesen, wenn die Zuführöffnung 100 nicht von der Drainageschicht 40 abgedeckt wird, um so das Absaugen von Wundexsudat ohne Beeinflussung durch die zugeführte Spüllösung zu gewährleisten und um zu verhindern, daß die zugeführte Spüllösung sofort wieder abgesaugt wird.

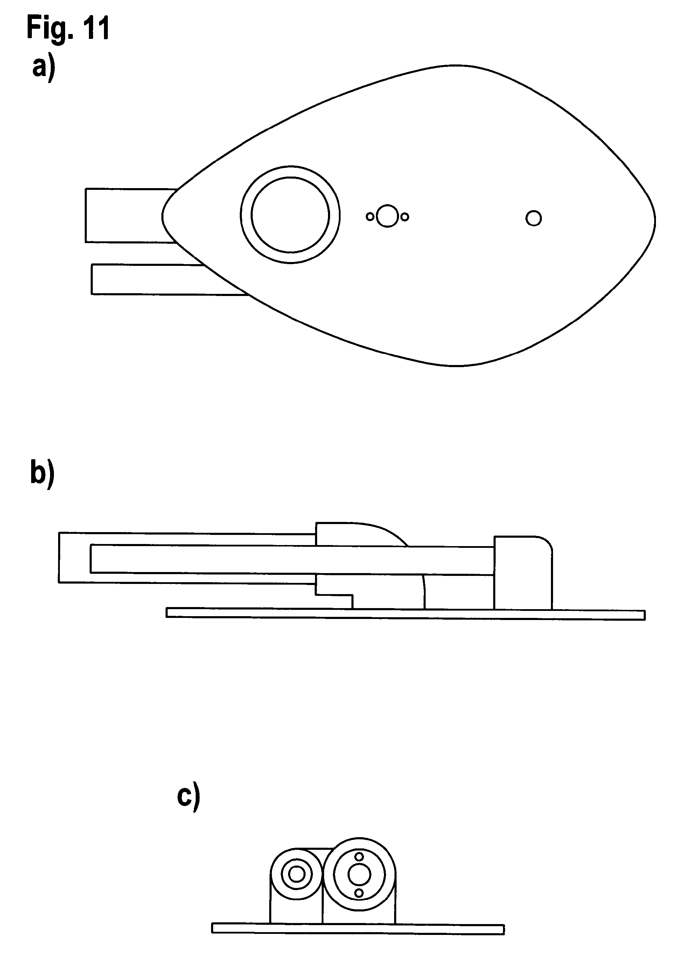

Die Ausführungsform gemäß Fig. 11 unterscheidet sich dadurch von der anhand der Fig. 9 erläuterten Ausführungsform, daß ein Dreilumenschlauch 132 zum Einsatz kommt, dessen Ausführung und Funktion der Ausführung gemäß Fig. 7 vergleichbar ist. Hierzu weist die Ausführung gemäß Fig. 11 einen Dreilumenschlauch zum Absaugen von Wundexsudat, Belüften des Wundbereichs und zur Druckmessung eine Belüftungsöffnung 80 sowie eine Zuführöffnung 100 auf. Dadurch kann ein optimales Wundmanagement gewährleistet werden.

Die anhand der Fig. 5 bis 11 erläuterten Ausführungsformen weisen anders als die anhand der in den Fig. 1 bis 4 erläuterten Ausführungsformen einen Anlagebereich mit einer von einer Kreisform abweichender Form, nämlich etwa rautenförmiger Form auf. Die Ecken der Rauten sind abgerundet. Durch die Rautenform wird eine Längsachse bereitgestellt, welche die Anordnung von Absaugöffnung, Belüftungsöffnung und Zuführöffnung auf einer Linie erlaubt.

Die Erfindung ist nicht auf die anhand der Zeichnung erläuterten Ausführungsbeispiele beschränkt. Vielmehr ist auch an den Einsatz von Sauganschlüssen mit einer von der Kreisscheibenform oder Rautenform abweichenden Form, wie etwa ovalen oder rechteckigen Sauganschlüssen, gedacht.

Ferner kann der Absaugschlauch auch auf die Rohrstutzen des Sauganschlusses aufgeschoben sein.

Wesentlich ist im Rahmen der Erfindung, daß zwischen der Anlagefläche des Sauganschlusses und dem Füllmaterial eine Drainageschicht der bspw. in der DE 10 2009 019 646 A1 angegebenen Art vorgesehen ist.

### BEZUGSZEICHENLISTE

- 10: Sauganschluß
- 11: Anlagebereich
- 12: Anlagefläche
- 14: Absaugöffnung
- 16, 16': Befestigungsbereich
- 20: Verbindungseinrichtung / Rohrstutzen
- 22: Anschlußbereich
- 24: Lumen (Unterdruck)
- 26: Belüftungslumen
- 28: Lumen (Druckmessung)
- 30: Absaugschlauch
- 32: Dreilumenschlauch
- 34: Lumen (Unterdruck)
- 36: Belüftungslumen
- 38: Lumen (Druckmessung)
- 40: Drainageschicht
- 50: Füllmaterial
- 60: Abdeckeinrichtung / Abdeckfolie
- 70: Saugeinrichtung
- 72: Sammelbehälter
- 80: Belüftungsöffnung
- 82: antibakterielles Filter
- 100: Zuführöffnung
- 110: Rohrstutzen
- 114: Dosierelement
- 130: Zuführschlauch
- 132: Dreilumenschlauch

## Patentansprüche

1. Wundversorgungsanordnung mit einem zum Füllen der Wunde ausgelegten Füllmaterial (50) und einem zum Absaugen von Wundexsudat aus dem Füllmaterial (50) ausgelegten und auf der dem Wundgrund abgewandten Seite außerhalb des Füllmaterials (50) anzuordnenden Sauganschluß (10), **gekennzeichnet durch** eine zwischen dem Füllmaterial (50) und dem Sauganschluß (10) angeordnete Drainageschicht (40) zum Einleiten des aus dem Füllmaterial (50) abzusaugenden Exsudats in mindestens eine Absaugöffnung (14) des Sauganschlusses (10).

2. Wundversorgungsanordnung nach Anspruch 1, **dadurch gekennzeichnet, daß** die Absaugöffnung (14) einen flanschartigen Anlagebereich (11) mit einer im wesentlichen ebenen und an die Drainageschicht (40) anlegbaren Anlagefläche (12) des Sauganschlusses (10) durchsetzt.

3. Wundversorgungsanordnung nach Anspruch 1 oder 2, **dadurch gekennzeichnet, daß** auf der der Anlagefläche (12) abgewandten Seite des Sauganschlusses (10) eine Verbindungseinrichtung, wie etwa ein Rohrstutzen (20), zum Herstellen einer Verbindung zwischen der Absaugöffnung (14) und einem Absaugschlauch (30) vorgesehen ist.

4. Wundversorgungsanordnung nach einem der vorhergehenden Ansprüche, **dadurch gekennzeichnet, daß** die Drainageschicht (40) an der Anlagefläche (12) befestigt, insbesondere auf die Anlagefläche (12) aufgeklebt, angeschweißt, an die Anlagefläche (12) geklammert und/oder genäht ist.

5. Wundversorgungsanordnung nach einem der vorhergehenden Ansprüche, **dadurch gekennzeichnet, daß** der Sauganschluß (10), insbesondere in dem flanschartigen Anlagebereich (11), von einer zur Belüftung der Wunde dienenden Belüftungsöffnung (80) durchsetzt ist.

6. Wundversorgungsanordnung nach Anspruch 5, **dadurch gekennzeichnet, daß** der Belüftungsöffnung (80) ein antibakterielles Filter (82) zugeordnet ist.

7. Wundversorgungsanordnung nach einem der Ansprüche 3 bis 6, **dadurch gekennzeichnet, daß** der Absaugschlauch (32) ein Multilumenschlauch, insbesondere mit drei Lumen ist.

8. Wundversorgungsanordnung nach einem der vorhergehenden Ansprüche, **gekennzeichnet durch** eine zum Zuführen eines Wundversorgungsprodukts ausgelegte und vorzugsweise den Anlagebereich (11) durchsetzende Zuführöffnung (100).

9. Wundversorgungsanordnung nach Anspruch 8, **dadurch gekennzeichnet, daß** auf der der Anlagefläche (12) abgewandten Seite des Sauganschlusses (10) eine weitere Verbindungseinrichtung (110), wie etwa ein weiterer Rohrstutzen, zum Herstellen einer Verbindung zwischen der Zuführöffnung (100) und einem Zuführschlauch (130) angeordnet ist.

10. Wundversorgungsanordnung nach einem der vorhergehenden Ansprüche, **gekennzeichnet durch** eine weitere zwischen dem Wundgrund und dem Füllmaterial (50) angeordnete Kontaktschicht.

11. Wundversorgungsanordnung nach einem der vorhergehenden Ansprüche, **dadurch gekennzeichnet, daß** die Drainageschicht (40) und/oder die Kontaktschicht zwei etwa parallel zueinander verlaufende bahnförmige Elemente aufweist, zwischen denen ein Drainageraum gebildet ist, dessen Tiefe in einer sich senkrecht zu den bahnförmigen Elementen erstreckenden Tiefenrichtung eine Kapillarwirkung auf in dem Drainageraum aufgenommene Exsudate gewährleistet.

12. Wundversorgungsanordnung nach Anspruch 11, **dadurch gekennzeichnet, daß** die Tiefe des Drainageraums 5 mm oder weniger und mindestens 0,5 mm beträgt.

13. Wundversorgungsanordnung nach einem der vorhergehenden Ansprüche, **gekennzeichnet durch** eine an der die Wunde umgebenden Haut luftdicht festlegbare und zur Herstellung eines das Füllmaterial (50) enthaltenden luftdicht abgeschlossenen Raums dienende Abdeckeinrichtung, insbesondere Abdeckfolie (60).

14. Wundversorgungsanordnung nach einem der vorhergehenden Ansprüche, **dadurch gekennzeichnet, daß** die Drainageschicht (40) die Anlagefläche (12) nur teilweise bedeckt und ein die Drainageschicht (40) vorzugsweise umlaufender Befestigungsbereich (16) der Anlagefläche (12) auf die Abdeckeinrichtung (60) aufklebbar ist.

15. Wundversorgungsanordnung nach Anspruch 13, **dadurch gekennzeichnet, daß** die Drainageschicht (40) die Anlagefläche (12) vollständig bedeckt und die Abdeckeinrichtung (60) auf einen der Anlagefläche (12) abgewandten, die Verbindungseinrichtung (20) umlaufenden Befestigungsbereich (16') des Sauganschlusses (10) aufgeklebt ist.

16. Wundversorgungsanordnung nach einem der vorhergehenden Ansprüche, **dadurch gekennzeichnet, daß** das Füllmaterial (50) einen offenporigen Schaum und/oder Gaze aufweist.

17. Sauganschluß für eine Wundversorgungsanordnung nach einem der vorhergehenden Ansprüche.

18. Sauganschluß nach Anspruch 16, **dadurch gekennzeichnet, daß** auf einer Anlagefläche (12) des Sauganschlusses (10) eine Drainageschicht (40) befestigt, insbesondere aufgeklebt, angeschweißt, an den Sauganschluß (10) geklammert und/oder genäht ist.

19. Sauganschluß nach Anspruch 17 oder 18, mit einem von einer Absaugöffnung (14) durchsetzten, insbesondere flanschartigen Anlagebereich (11), **dadurch gekennzeichnet, daß** der Anlagebereich (11) zusätzlich von einer Belüftungsöffnung (80) und/oder einer zum Zuführen eines Wundversorgungsprodukts dienenden Zuführöffnung (100) durchsetzt ist.
